# DEMANDE DE BREVET EUROPEEN

(11) **EP 2 363 119 A1**
(43) Date de publication de la demande: **07.09.2011**
(21) Numéro de dépôt: 10305227.0
(22) Date de dépôt: 05.03.2010
(51) Int. Cl.: A61K 31/13, A61K 31/592, A61K 31/593, A61K 45/06, A61P 25/00

(54) **Nouvelle composition pharmaceutique utile pour le traitement des personnes affectées par des maladies neurodégénératives ou neurovasculaires**

(71) Demandeur: Centre Hospitalier Universitaire d'Angers, 49333 Angers (FR); UNIVERSITE D'ANGERS, 49035 Angers Cedex 01 (FR)
(72) Inventeur: Beauchet, Olivier, 49240, Avrillé (FR); Annweiler, Cédric, 49100, Angers (FR)
(74) Mandataire: Maureau, Philippe

(57) **Abrégé**

La présente invention a pour objet une composition pharmaceutique utile pour le traitement de maladies neurodégénératives ou neurovasculaires comprenant, à titre de principes actifs, de la mémantine et de la vitamine D.

## Description

La présente invention a pour objet une nouvelle composition pharmaceutique utile pour le traitement des maladies neurodégénératives ou neurovasculaires.

Les maladies neurodégénératives sont des maladies conduisant généralement à une détérioration du fonctionnement des cellules nerveuses, en particulier les neurones, voire à leur mort cellulaire. La conséquence pour le malade est donc une altération progressive, et dans la plupart des cas irréversible, des fonctions nerveuses pouvant conduire au décès de la personne atteinte par ce type de maladies.

Ces maladies affectent le fonctionnement du système nerveux, et plus particulièrement du cerveau, de façon progressive au cours de son évolution. Cette évolution peut être plus ou moins longue, pouvant aller de quelques semaines à plusieurs années.

En fonction des régions du système nerveux atteintes par la maladie, les troubles observés peuvent concerner la motricité ou toute ou partie des fonctions cognitives telles que le langage, la mémoire, la perception ou d'autres fonctions cognitives.

Le glutamate est la forme anionique de l'acide glutamique indispensable au bon fonctionnement des cellules nerveuses du système nerveux central, et plus précisément du cerveau. Il s'agit en effet de l'un des 20 acides-α-aminés naturels constituant les protéines des cellules nerveuses. Par ailleurs, il est le neurotransmetteur excitateur le plus répandu dans le système nerveux central. Il agit en se fixant sur les récepteurs membranaires glutamatergiques post-synaptiques de type ionotrope (N-méthyl-D-aspartate (NMDA), AMPA, kaïnate) ou métabotrope. Les synapses glutamatergiques représentent 50% des synapses du système nerveux central. Cette transmission nerveuse glutamatergique a été associée d'un point de vue clinique aux capacités d'apprentissage et de mémorisation.

En cas de libération excessive dans la fente synaptique, le glutamate est à l'origine d'un message excitateur neurotoxique (connu sous le nom d'excitotoxicité neuronale) conduisant à la mort neuronale. Un tel dysfonctionnement de la neurotransmission glutamatergique est suspecté d'être impliqué dans la physiopathologie de maladies neurodégénératives telles que la maladie d'Alzheimer.

En effet, l'excès de glutamate libéré dans la fente synaptique est à l'origine d'une surexcitation des récepteurs glutamatergiques post-synaptiques aux premiers rangs desquels on retrouve les récepteurs NMDA, avec comme conséquence une entrée excessive de calcium dans le neurone post-synaptique aboutissant à la mort neuronale. *In vitro,* l'intensité de ce stimulus initial induit deux mécanismes distincts de mort neuronale :
- l'influx calcique intra-neuronal est très important et dépasse les capacités de stockage (situation A). Dans ce cas, l'excitotoxicité est immédiate et aboutit à une perte du potentiel de membrane mitochondriale et de la charge énergétique et à la faillite de l'homéostasie interne (gonflement passif du noyau et de la cellule neuronale, atteinte des organites cytoplasmiques, libération du contenu intracellulaire et intranucléaire dans le milieu extracellulaire). Il s'agit d'un phénomène de nécrose neuronale immédiate.
- l'influx calcique intra-neuronal est modérément excessif (situation B). Dans ce cas, l'excitotoxicité est retardée et liée à un stress oxydatif : l'entrée excessive de calcium dans le neurone cause une cascade d'évènements parmi lesquels l'activation de la nitric oxyde synthase et la synthèse d'oxyde nitrique (NO), ou la stimulation de la phospholipase A2, ou encore l'entrée de calcium dans la mitochondrie entraînant la génération d'anion superoxyde (O₂⁻). NO peut interagir avec O₂⁻ pour former le peroxynitrite (OONO⁻). De ces réactions d'oxydation et de réduction résultent des radicaux libres qui induisent des dommages neuronaux dose-dépendants (lésions de l'acide désoxyribonucléique (ADN), des lipides membranaires par peroxydation lipidique, des protéines cellulaires essentielles et inactivation enzymatique). Ces modifications s'accompagnent *in vitro* d'une rétraction cellulaire, d'une relocalisation des organites, d'une condensation de la chromatine, d'une fragmentation nucléaire, et de la production de corps apoptotiques contenant des fragments de cytoplasme et de noyau : il s'agit d'un phénomène d'apoptose neuronale retardée (mort cellulaire programmée en réponse au stimulus toxique).

La conséquence clinique de ces réactions en chaîne pour les neurones glutamatergiques est la perte des capacités d'apprentissage et de mémoire caractérisant les syndromes démentiels.

L'une des maladies neurodégénératives les plus fréquemment rencontrées et diagnostiquées est la maladie d'Alzheimer.

Dans la plupart des maladies neurodégénérative, comme par exemple la maladie d'Alzheimer, on distingue quatre stades distincts dans l'évolution de la maladie :
1) le stade pré-démentiel ;
2) le stade léger (ou premier stade de la démence) ;
3) le stade modéré (ou deuxième stade de la démence) ; et
4) le stade avancé (ou troisième stade de la démence).

La maladie d'Alzheimer débute habituellement par des troubles de la mémoire. Néanmoins, cette maladie peut également se manifester par l'apparition d'autres symptômes tels qu'une dépression, une perte d'indépendance fonctionnelle, des chutes répétées, un amaigrissement ou encore des troubles du comportement.

A un stade plus évolué (à partir du deuxième stade de la démence), d'autres troubles cognitifs apparaissent progressivement : altération du langage, des praxies, de la motricité ou encore de la communication.

Actuellement, il n'existe aucun traitement permettant de guérir les patients atteints de maladies neurodégénératives telles que la maladie d'Alzheimer, ni même qui permette de stopper l'évolution de la maladie. Quelques médicaments peuvent cependant retarder l'évolution de la maladie en atténuant la détérioration ou la perte de mémoire, du langage ou du raisonnement. Parmi les médicaments les plus prescrits pour tenter de ralentir l'évolution de ces maladies, on trouve notamment la mémantine, un antagoniste des récepteurs NMDA non compétitif, de faible affinité, voltage-dépendant. L'efficacité de ce médicament a été établie par des essais en double aveugle contre placebo. Ils n'ont pas d'effet immédiat, mais après 3 à 6 mois d'utilisation, les patients qui ont reçu le traitement ont des fonctions cognitives et une autonomie meilleures que les patients qui ont reçu le placebo. Autrement dit, par ce traitement, le déclin est retardé mais pas traité. En effet, la faible affinité et la cinétique rapide de retrait de la mémantine au niveau du canal des récepteurs NMDA préserve la fonction physiologique de ces récepteurs qui restent activables par le glutamate relâché suite à la dépolarisation des neurones présynaptiques. Néanmoins, en se liant aux récepteurs NMDA avec plus d'affinité que les ions magnésium Mg²⁺, la mémantine est capable d'inhiber l'influx prolongé d'ions calcium Ca²⁺. Il en résulte une protection des neurones glutamatergiques liée à l'évitement de la situation A évoquée ci-dessus et des phénomènes de nécrose neuronale consécutifs à l'influx excessif et prolongé de calcium dans la cellule. Les patients ayant une maladie d'Alzheimer et prenant de la mémantine se retrouvent donc dans la situation B discutée ci-dessus, c'est-à-dire que la mort neuronale par excitotoxicité immédiate est limitée mais qu'il existe toujours un influx modérément excessif de calcium dans le neurone post-synaptique, conduisant à un stress oxydatif des espèces réactives de l'oxygène, du nitrogène et des radicaux libres qui aboutit à la mort neuronale par apoptose.

Ainsi, la mémantine, si elle permet un traitement symptomatique de la maladie d'Alzheimer, c'est-à-dire un traitement permettant de ralentir l'évolution de la maladie, ne permet pas un traitement préventif ou curatif de celle-ci, qui permettrait d'empêcher son apparition ou de soigner/traiter celle-ci.

La vitamine D est une hormone stéroïde qui se fixe sur des récepteurs stéroïdes de la vitamine D (VDR) présents au niveau des neurones et des cellules gliales du système nerveux central dont l'hippocampe, l'hypothalamus, le cortex ou le sous-cortex. La 1,25-OHD (forme active de la vitamine D) a précisément démontré *in vitro* des qualités de neuroprotection contre la toxicité du glutamate grâce à des effets anti-oxydants. Cette action de détoxification a été décrite en 2001 sur des cultures de cellules mésencéphaliques de rat par Ibi et al. dans un article intitulé Protective effects of 1 alpha,25-(OH)(2)D(3) against the neurotoxicity of glutamate and reactive oxygen species in mesencephalic culture, Neuropharmacology 2001; 40: 761-771. Néanmoins, il n'a jamais été décrit ni suggéré que la vitamine D permettrait un traitement préventif ou curatif des maladies neurodégénératives telles que la maladie d'Alzheimer, c'est-à-dire un traitement permettant d'empêcher l'apparition de la maladie ou de soigner/traiter celle-ci.

Ainsi, à la date de la présente invention, il n'existe aucun traitement guérissant les patients atteints de maladies neurodégénératives, ni même permettant de stopper leur évolution.

D'autre part, les maladies neurodégénératives décrites ci-dessus peuvent être associées à des atteintes vasculaires ischémiques ou hémorragiques, définissant ainsi des affections mixtes, aggravant les troubles des fonctions cognitives. En dehors d'une atteinte dite mixte, les atteintes vasculaires peuvent être isolées et à l'origine de troubles des fonctions cognitives allant du stade pré-démentiel au stade de démence sévère.

Or, il a été trouvé de façon toute à fait surprenante que l'association médicamenteuse mémantine / vitamine D apporte une solution efficace au problème de la mort neuronale et de la perte de fonction cognitive au cours du vieillissement pathologique cérébral, et permet donc de soigner ou stopper l'évolution de maladies neurodégénératives et ce, quel que soit le stade de la maladie. De plus, il a également été trouvé que cette association permettait également de prévenir et traiter efficacement les maladies neurovasculaires et ce, quel que soit le stade de la maladie.

La présente invention a donc pour objet une composition pharmaceutique comprenant deux principes actifs choisis comme étant :
- la mémantine ; et
- la vitamine D.

La composition pharmaceutique selon l'invention permet la prévention et le traitement des maladies neurodégénératives chez l'être humain quel que soit le stade de la maladie, y compris au stade pré-démentiel. La composition pharmaceutique selon l'invention permet également la prévention et le traitement des maladies neurovasculaires chez l'être humain.

Dans le cadre de la présente invention :
- on entend par « maladie neurodégénérative » l'ensemble des affections cérébrales neurodégénératives, la maladie d'Alzheimer, les maladies apparentées à la maladie d'Alzheimer, les démences fronto-temporales et apparentées, les démences associées à la maladie de Parkinson, la maladie à corps de Lewy, les démences mixtes combinant une affection neurodégénérative et une atteinte vasculaire cérébrale, quel que soit le stade du syndrome démentiel et l'âge de début des troubles ;
- on entend par « maladie neurovasculaire » l'ensemble des affections cérébrales neurovasculaires, et les démences neurovasculaires isolées, quel que soit le stade du syndrome démentiel et l'âge de début des troubles ;
- on entend par « sel pharmaceutiquement acceptable » d'un principe actif tout sel d'addition dudit principe actif avec un acide minéral ou organique par action d'un tel acide au sein d'un solvant organique ou aqueux tel qu'un alcool, une cétone, un éther ou un solvant chloré, et qui soit acceptable d'un point de vue pharmaceutique ;
- on entend par « dérivé pharmaceutiquement acceptable » d'un principe actif tout « prodrogue » **ou** « métabolite » **dudit principe actif,** ainsi que leur sel pharmaceutiquement acceptable ;
- **on entend par** «prodrogue» **d'un principe actif tout composé** dont la biotransformation dans l'organisme aboutit audit principe actif ;
- on entend par « métabolite » d'un principe actif tout produit intermédiaire résultant de la transformation dudit principe actif dans l'organisme lors d'un processus métabolique ;
- on entend par « administration journalière » une administration une fois par jour ou une administration une fois par 24 heures ;
- on entend par « administration hebdomadaire » une administration une fois par semaine ou une fois tous les 7 jours ;
- on entend par « administration bimensuelle » une administration deux fois par mois ou une administration une fois tous les 14 ou 15 jours ;
- on entend par « administration mensuelle » une administration une fois par mois ou une administration une fois tous les 28, 29, 30 ou 31 jours ;
- on entend par « administration trimestrielle» une administration une fois par trimestre ou une administration une fois tous les 3 mois ;
- on entend par « administration semestrielle» une administration une fois par semestre ou une administration une fois tous les 6 mois ;
- on entend par « calendrier continu » le traitement thérapeutique continu d'un patient, de façon illimitée et non séquencée ou espacée dans le temps, c'est-à-dire sans interruption de traitement ;
- la mémantine désigne la 3,5-diméthyl-tricyclo[3.3.1.1]décyl amine, ainsi que ses sels ou dérivés pharmaceutiquement acceptables ;
- la vitamine D désigne la vitamine D₂ (ergocalciférol), la vitamine D₃ (ou cholécalciférol) **ou un mélange des deux**, ainsi que ses sels ou dérivés pharmaceutiquement acceptables ; et
- UI désigne une Unité Internationale, étant entendu que 1 UI de vitamine D équivaut à 0.025µg d'ergocalciférol/cholécalciférol.

La composition pharmaceutique selon la présente invention contient les principes actifs en quantité suffisante pour assurer l'effet thérapeutique souhaité, c'est-à-dire le traitement des maladies neurodégénératives permettant de stopper ou faire reculer l'évolution de la maladie chez le patient traité, ainsi que la prévention ou le traitement des maladies neurovasculaires.

De préférence, les quantités suivantes de principe actif sont utilisées pour préparer la composition pharmaceutique selon l'invention :
- de 1 à 40 mg de mémantine, de préférence de 5 à 20 mg de mémantine;
- de 200 à 10000 UI de Vitamine D, de préférence de 400 à 5000 UI de vitamine D, de préférence encore de 800 à 1500 UI de vitamine D.

La composition pharmaceutique selon la présente invention peut être formulée sous toute forme galénique nécessaire à son administration. En particulier, s'agissant d'administration par voie orale, les compositions selon la présente invention peuvent être formulées sous forme de comprimés enrobés ou non, effervescents, solubles, orodispersibles, gastrorésistants ou à libération modifiée ; de dragées ; de capsules à enveloppe dure (ou gélules) ; de capsules à enveloppe molle ; de granules ; de granulés ; de pilules ; de pastilles. S'agissant d'administration par voie systémique, la composition selon l'invention peut être formulée sous forme de poudre lyophilisée stérile pour injection. Les compositions pharmaceutiques selon la présente invention pourront donc comprendre, en plus des principes actifs, tout adjuvant de formulation pharmaceutiquement acceptable, connu de l'homme du métier et qui est nécessaire à la préparation de la composition pharmaceutique sous la forme souhaitée.

Certaines compositions pharmaceutiques selon la présente invention peuvent être administrées à tout patient atteint ou susceptible d'être atteint par une maladie neurodégénérative ou neurovasculaire.

Ainsi, la présente invention a également pour objet une composition pharmaceutique telle que définie précédemment pour le traitement préventif ou curatif des maladies neurodégénératives. Préférentiellement, la présente invention a également pour objet une composition pharmaceutique telle que définie précédemment pour le traitement préventif ou curatif de l'ensemble des affections cérébrales neurodégénératives, de la maladie d'Alzheimer, des maladies apparentées à la maladie d'Alzheimer, des démences fronto-temporales et apparentées, des démences associées à la maladie de Parkinson, de la maladie à corps de Lewy ou encore des démences mixtes combinant une affection neurodégénérative et une atteinte vasculaire cérébrale, quel que soit le stade du syndrome démentiel et l'âge de début des troubles. Préférentiellement encore, la présente invention permet le traitement préventif ou curatif de l'une des maladies ci-dessus chez l'être humain à un stade pré-démentiel.

D'autre part, la présente invention a également pour objet une composition pharmaceutique telle que définie précédemment pour le traitement préventif ou curatif des maladies neurovasculaires. Préférentiellement, la présente invention a également pour objet une composition pharmaceutique telle que définie précédemment pour le traitement préventif ou curatif de l'ensemble des affections cérébrales neurovasculaires, et les démences neurovasculaires isolées chez l'être humain, quel que soit le stade du syndrome démentiel et l'âge de début des troubles. Préférentiellement encore, la présente invention permet le traitement préventif ou curatif de l'une des maladies ci-dessus chez l'être humain à un stade pré-démentiel.

La composition pharmaceutique selon l'invention peut être administrée à tout moment de la journée, de préférence à la même heure chaque jour, avant, pendant ou après les repas, sans que cela n'influe sur l'efficacité du traitement.

D'autre part, la composition pharmaceutique selon la présente invention peut être administrée de façon journalière.

La composition selon la présente invention peut être administrée selon un calendrier continu.

Un autre objet de la présente invention concerne également l'utilisation d'une composition pharmaceutique telle que définie précédemment pour la préparation d'un médicament destiné au traitement préventif ou curatif des maladies neurodégénératives chez l'être humain. De préférence, la présente invention a pour objet l'utilisation d'une composition pharmaceutique telle que définie précédemment pour la préparation d'un médicament destiné au traitement préventif ou curatif de l'ensemble des affections cérébrales neurodégénératives, de la maladie d'Alzheimer, des maladies apparentées à la maladie d'Alzheimer, des démences fronto-temporales et apparentées, des démences associées à la maladie de Parkinson, de la maladie à corps de Lewy ou encore des démences mixtes combinant une affection neurodégénérative et une atteinte vascualaire cérébrale, quel que soit le stade du syndrome démentiel et l'âge de début des troubles. Préférentiellement encore, la présente invention a pour objet l'utilisation d'une composition pharmaceutique telle que définie précédemment pour la préparation d'un médicament destiné au traitement préventif ou curatif de l'une des maladies ci-dessus à un stade pré-démentiel.

D'autre part, la présente invention a également pour objet également l'utilisation d'une composition pharmaceutique telle que définie précédemment pour la préparation d'un médicament destiné au traitement préventif ou curatif des maladies neurovasculaires chez l'être humain. De préférence la présente invention a également pour objet l'utilisation d'une composition pharmaceutique telle que définie précédemment pour la préparation d'un médicament destiné au traitement préventif ou curatif des affections cérébrales neurovasculaires, et les démences neurovasculaires isolées chez l'être humain, quel que soit le stade du syndrome démentiel et l'âge de début des troubles. Préférentiellement encore, la présente invention a pour objet l'utilisation d'une composition pharmaceutique telle que définie précédemment pour la préparation d'un médicament destiné au traitement préventif ou curatif de l'une des maladies ci-dessus à un stade pré-démentiel.

Un autre objet de la présente invention concerne également l'utilisation d'une composition pharmaceutique telle que définie précédemment pour la préparation d'un médicament destiné au traitement préventif ou curatif des maladies neurodégénératives ou neurovasculaires chez l'être humain, ledit médicament étant administré de façon journalière et continue.

La présente invention a également pour objet une méthode de traitement préventif ou curatif des maladies neurodégénératives chez un être humain par l'administration d'une composition pharmaceutique telle que de définie précédemment. De préférence, la présente invention a pour objet une méthode de traitement préventif ou curatif de de la maladie d'Alzheimer, des maladies apparentées à la maladie d'Alzheimer, des démences fronto-temporales et apparentées, des démences associées à la maladie de Parkinson, de la maladie à corps de Lewy ou encore des démences mixtes combinant une affection neurodégénérative et une atteinte vasculaire cérébrale, quel que soit le stade du syndrome démentiel et l'âge de début des troubles par l'administration d'une composition pharmaceutique telle que de définie précédemment. Préférentiellement encore, la présente invention a pour objet une méthode de traitement préventif ou curatif de l'une des maladies ci-dessus à un stade pré-démentiel par l'administration d'une composition pharmaceutique telle que de définie précédemment.

D'autre part, la présente invention a également pour objet une méthode de traitement préventif ou curatif des maladies neurovasculaires chez un être humain par l'administration d'une composition pharmaceutique telle que de définie précédemment. Préférentiellement, la présente invention a également pour objet une méthode de traitement préventif ou curatif des affections cérébrales neurovasculaires, et les démences neurovasculaires isolées chez l'être humain par l'administration d'une composition pharmaceutique telle que de définie précédemment, quel que soit le stade du syndrome démentiel et l'âge de début des troubles. Préférentiellement encore, la présente invention a pour objet une méthode de traitement préventif ou curatif de l'une des maladies ci-dessus à un stade pré-démentiel par l'administration d'une composition pharmaceutique telle que de définie précédemment.

La présente invention a également pour objet une méthode de traitement préventif ou curatif des maladies neurodégénératives ou neurovasculaires chez un être humain par l'administration journalière continue.

Les **deux principes actifs constituant** le nouveau traitement des maladies neurodégénératives selon l'invention peuvent être administrés sous la forme d'une composition pharmaceutique unitaire comprenant les deux principes actifs permettant une administration de ladite composition au patient en une seule prise.

Néanmoins, une administration séparée des principes actifs constitutifs du nouveau traitement selon l'invention peut également être envisagée. Ainsi, la présente invention a également pour objet un produit pharmaceutique contenant :
- la mémantine ; et
- la vitamine D ;
comme produit de combinaison (ou kit pharmaceutique) pour une administration simultanée, séparée ou étalée dans le temps.

De préférence, le produit pharmaceutique selon la présente invention est utilisé dans le cadre du traitement des maladies neurodégénératives chez l'être humain telles que l'ensemble des affections cérébrales neurodégénératives, la maladie d'Alzheimer, les maladies apparentées à la maladie d'Alzheimer, les démences fronto-temporales et apparentées, les démences associées à la maladie de Parkinson, la maladie à corps de Lewy, les démences mixtes combinant une affection neurodégénérative et une atteinte vasculaire cérébrale, quel que soit le stade du syndrome démentiel et l'âge de début des troubles.

Le produit pharmaceutique selon la présente invention peut également être utilisé est utilisé dans le cadre du traitement des maladies neurovasculaires chez l'être humain telles que les affections cérébrales neurovasculaires, et les démences neurovasculaires isolées, quel que soit le stade du syndrome démentiel et l'âge de début des troubles.

Le produit pharmaceutique selon la présente invention peut également être utilisé dans le cadre du traitement préventif ou curatif de l'une des maladies ci-dessus chez l'être humain à un stade pré-démentiel.

De préférence, les quantités journalières suivantes de principe actif sont utilisées pour préparer la composition pharmaceutique selon l'invention :
- de 1 à 40 mg de mémantine, de préférence de 5 à 20 mg de mémantine;
- de 200 à 10000 UI de Vitamine D, de préférence de 400 à 5000 UI de vitamine D, de préférence encore de 800 à 1500 UI de vitamine D.

Le produit pharmaceutique selon l'invention peut bien entendu être administré selon l'un des schémas d'administration définis précédemment.

Selon un schéma d'administration préféré du produit pharmaceutique selon l'invention :
- la forme posologique unitaire contenant la mémantine est administrée de façon journalière ; et
- la forme posologique unitaire contentant la vitamine D est administrée de façon hebdomadaire, bimensuelle, mensuelle, trimestrielle ou semestrielle.

Bien entendu, la quantité de principe actif contenue dans chaque forme posologique unitaire sera ajustée en fonction de la fréquence d'administration envisagée et de la quantité journalière de principe actif devant être administrée. A titre d'exemple, on peut ainsi citer les formes posologiques unitaires contenant :
- de 200 à 10000 UI de Vitamine D, de préférence de 400 à 5000 UI de vitamine D, de préférence encore de 800 à 1500 UI de vitamine D, pour une administration journalière ; et
- de 1400 à 70000 UI de Vitamine D, de préférence de 2800 à 35000 UI de vitamine D, de préférence encore de 5600 à 10500 UI de vitamine D, pour une administration hebdomadaire.

## Revendications

1. Composition pharmaceutique comprenant deux principes actifs choisis comme étant :
- la mémantine ; et
- la vitamine D.

2. Composition pharmaceutique selon la revendication 1, **caractérisée en ce que** qu'elle contient de 1 à 40 mg de mémantine.

3. Composition pharmaceutique selon la revendication 1 ou 2, **caractérisée en ce que** qu'elle contient de 200 à 10000 UI de vitamine D.

4. Composition pharmaceutique selon l'une des revendications 1 à 3 pour le traitement préventif ou curatif des maladies neurodégénératives.

5. Composition pharmaceutique selon la revendication 4, **caractérisée en ce que** la maladie neurodégénérative est choisie parmi l'ensemble des affections cérébrales neurodégénératives, la maladie d'Alzheimer, les maladies apparentées à la maladie d'Alzheimer, les démences fronto-temporales et apparentées, les démences associées à la maladie de Parkinson, la maladie à corps de Lewy, les démences mixtes combinant une affection neurodégénérative et une atteinte vasculaire cérébrale.

6. Composition pharmaceutique selon l'une des revendications 1 à 3 pour le traitement préventif ou curatif des maladies neurovasculaires.

7. Composition pharmaceutique selon l'une des revendications 4 à 6, **caractérisée en ce que** le traitement de la maladie au stade pré-démentiel.

8. Composition pharmaceutique selon l'une des revendications 1 à 7, ladite composition pharmaceutique étant administrée de façon journalière et continue.

9. Produit pharmaceutique contenant :
- la mémantine ; et
- la vitamine D ;
comme produit de combinaison pour une administration simultanée, séparée ou étalée dans le temps.

10. Produit pharmaceutique selon la revendication 9, utilisé dans le cadre du traitement des maladies neurodégénératives chez l'être humain.

11. Produit pharmaceutique selon la revendication 10, **caractérisé en ce que** la maladie neurodégénérative est choisie parmi l'ensemble des affections cérébrales neurodégénératives, la maladie d'Alzheimer, les maladies apparentées à la maladie d'Alzheimer, les démences fronto-temporales et apparentées, les démences associées à la maladie de Parkinson, la maladie à corps de Lewy, les démences mixtes combinant une affection neurodégénérative et une atteinte vasculaire cérébrale.

12. Produit pharmaceutique selon la revendication 9, utilisé dans le cadre du traitement des maladies neurovasculaires chez l'être humain.
